# EUROPEAN PATENT APPLICATION

(11) **EP 4 715 051 A1**
(43) Date of publication of application: **25.03.2026**
(21) Application number: 24806679.7
(22) Date of filing: 17.05.2024
(51) Int. Cl.: C12N 15/09, A61K 31/713, A61P 31/14

(54) **USE OF SMALL NUCLEIC ACID IN PREPARATION OF DRUG FOR TREATING FELINE INFECTIOUS PERITONITIS**

(30) Priority: 17.05.2023 CN 202310557391
(71) Applicant: Nanjing Huili Biotechnology Co., Ltd., Nanjing, Jiangsu 210018 (CN)
(72) Inventor: HU, Xiaoyun, Nanjing, Jiangsu 210018 (CN); DU, Manman, Nanjing, Jiangsu 210018 (CN)
(74) Representative: Lavoix
(86) International application number: PCT/CN2024/094067
(87) International publication number: WO 2024/235330

(57) **Abstract**

Provided are a small nucleic acid for inhibiting feline infectious peritonitis virus (FIPV) and a pharmaceutical composition containing the small nucleic acid. Provided is a small RNA having a core sequence of GCCGGG. The small RNA can bind to multiple FIPV gene sites, can directly target FIPV *in vitro,* can achieve an antiviral effect in vivo in an animal, and has prospects for application in the veterinary field.

## Description

### TECHNICAL FIELD

The present invention relates to the field of bio-pharmaceuticals. Specifically, the present invention relates to the use of small nucleic acids in the preparation of drugs for treating feline infectious peritonitis.

### BACKGROUND

Feline Infectious Peritonitis (FIP) is known as a fatal disease in cats with a 100% mortality rate. It is characterized by the development of peritonitis and ascites, has a worldwide distribution, occurs in cats of all ages and different genders, and shows no seasonal pattern in its onset. Current researches widely support the internal mutation theory for the pathogenesis of FIP, i.e., the virus acquires mutations within individual cats and transforms into FIPV. FIPV can infect feline monocytes and then replicates in monocytes continuously and efficiently. The infected monocytes produce cytokines, adhesion molecules and enzymes, leading to visible vascular and perivascular lesions of FIP.

The pathological characteristics of FIP is systemic vasculitis and/or granuloma pyogenicum, and its clinical symptoms depend on the organs affected. Common lesion sites for FIP include serosa, kidneys, mesenteric lymph nodes, brain, eyes, liver, spleen, and lungs, etc, resulting clinical symptoms including ascites, dyspnea, uveitis, ataxia, and seizures. Affected cats may also exhibit non-specific symptoms such as persistent fever, lethargy, anorexia, jaundice, scrotal enlargement, and weight loss. Based on symptoms, FIP is categorized into two types: the effusive ("wet") and the non-effusive ("dry"). Effusive FIP involves serositis and fluid exudation, with a survival time ranging from days to weeks. Non-effusive FIP primarily involves perivascular vasculitis, with a survival time of weeks to months. Some infected cats may present with a dry-wet FIP, whose post-mortem examination reveals lesions on both serosa and in blood vessels. Both types of FIP are fatal; however, the effusive type is more common than the non-effusive type, accounting for approximately 60%-70% of cases, and progresses more rapidly.

Clinical diagnosis of FIP is challenging. Reliable diagnosis can be obtained based on clinical signs, combined with medical history and laboratory tests. Lab results of blood routine and biochemistry may present abnormalities including: mild to moderate non-regenerative anemia, microcytosis, lymphopenia, neutrophilia (with or without left shift), hyperbilirubinemia, hyperglobulinemia, hypoalbuminemia, and an albumin-to-globulin (A:G) ratio of less than 0.8. Effusive FIP involves fluid accumulation. Laboratory analysis on aspirated abdominal effusion, typically a yellow, clear or cloudy, viscous fluid, reveals high protein content (>35 g/L) and low nucleated cell count (<5,000 nucleated cells/µL). Albumin and globulin can be detected in the effusion, and an A:G ratio of less than 0.4 is highly suggestive of FIP. Furthermore, virus isolation and identification can be attempted by inoculating effusion or blood into feline embryonic lung cell cultures, or through neutralization tests, immunofluorescence assays, ELISA, or PCR. Among these, immunohistochemistry is considered the gold standard for FIP detection due to its high positive detection rate, although false negatives can occur due to the sample selected. Commercial antibody test kits for detecting FCoV antibodies in serum/effusion/cerebrospinal fluid are available for FIP diagnosis, but their clinical use is not recommended because positive results are frequently found in healthy FCoV-infected cats, while negative results can occur in cats with FIP. Reverse Transcription-Polymerase Chain Reaction (RT-PCR) can detect FCoV RNA in various clinical samples but must also be used cautiously, with results interpreted in combination with the cat's medical history and clinical symptoms. Sequencing to detect gene mutations is also possible; however, as these gene mutations are not specific to FIPV, gene sequencing alone outside of a RT-PCR context is not recommended for clinical diagnosis.

Research on FIP treatment has a fairly long history, with literature dating back to the 1960s, reporting the use of corticosteroids, penicillin, and streptomycin in cats highly suspected of having FIP. Numerous studies have reported on FIP treatments, whose investigated agents include glucocorticoids, ribavirin, interferon-α, interferon-ω, and tylosin, and etc. Current FIP treatment research primarily focuses on immunosuppressants, antiviral drugs, and immunomodulators. Immunosuppressants, mainly glucocorticoids, cyclophosphamide, chloramphenicol, and salicylic acid, can suppress FIP-related inflammation and may prolong the survival time of infected cats, although no definitive studies have yet proven their efficacy. Immunomodulators are widely used in FIP-infected cats, common examples include tylosin, interferon-α, and interferon-ω. In clinical practice, immunomodulators are generally not used alone, rather combined with immunosuppressive drugs.

The latest research focus is nucleoside analogues. Nucleoside analogues that have been reported for FIP treatment research include GC376 and GS-441524. GC376 is a 3C protease inhibitor. Coronaviruses possess a 3C protease, a cysteine protease responsible for cleaving most of the viral polyprotein precursors and the processing of mature proteins. The mechanism of the protease inhibitor GC376 involves blocking of viral protease cleavage, thereby inhibiting viral replication. While published literature confirms the efficacy of GC-376 in treating experimentally induced FIP, studies also indicate that GC376 treatment requires long cycles, faces a risk of relapse, and has a relatively low cure rate. Whether a cat can be cured still depends heavily on its own immune resistance, thus GC376 cannot produce fully reliable therapeutic outcomes. GS-441524 is a nucleoside triphosphate competitive inhibitor. It can be activated by cellular kinases to a nucleoside monophosphate and subsequently to a biologically active nucleoside triphosphate (NTP) metabolite. Viral RNA replication requires the participation of natural nucleoside triphosphates. The biologically active NTP metabolite can competitively act as a substrate for the viral RNA-dependent RNA polymerase and as an RNA chain terminator, thereby inhibiting viral replication. However, this drug is still under development, and research regarding its use in treating FIP remains immature.

Therefore, there is a pressing need in the field for an effective and safe method for preparing drugs for the treatment of feline infectious peritonitis.

### SUMMARY OF INVENTION

The present invention seeks to provide an effective and safe drug for the treatment of FIP.

In a first aspect of the invention, provided is a small RNA, or an RNA or DNA complementary or reverse-complementary thereto, or a modified derivative thereof, wherein, said small RNA comprises a core sequence of GCCGGG, and the length of said small RNA is 18-30 nucleotides,
provided that the small RNA sequence is not the sequence shown in SEQ ID NO.54.

In another preferred embodiment, said small RNA is non-natural.

In another preferred embodiment, said small RNA comprises the artificial synthesized or the recombinantly expressed.

In another preferred embodiment, said small RNA is single or double-stranded.

In another preferred embodiment, said small RNA has a sequence construct from the 5' to the 3' end as shown in Formula (I) below:

(P)n-R-(Q)m (I);

wherein,
P is selected from A, U, G or C;
R is the core sequence, which has the nucleotide sequence GCCGGG;
Q is selected from A, U, G or C;
and, said (P)n represents n number of P, with n being an integer selected from 0 to 4,
said (Q)m represents m number of Q, with m being an integer selected from 9 to 24.

In another preferred embodiment, said small RNA has a length of 19-27 nucleotides.

In another preferred embodiment, said n is 0, 1, 2, 3 or 4.

In another preferred embodiment, said m is an integer selected from 12-19.

In another preferred embodiment, said small RNA binds to FIPV gene.

In another preferred embodiment, the binding energy of said small RNA with FIPV gene is less than -15.0kcal/mol, preferably less than -20.0kcal/mol, and more preferably less than -24.0kcal/mol.

In another preferred embodiment, said FIPV gene is selected from the group consisting of: S (spike protein), M, ORF 1a/1b, or the combination thereof.

In another preferred embodiment, said small RNA has a nucleotide sequence as shown in any one selected from SEQ ID NOs.1-53.

In another preferred embodiment, said small RNA has a nucleotide sequence as shown in SEQ ID NO.1, SEQ ID NO.9 or SEQ ID NO.37.

In another preferred embodiment, the modification of the modified derivative is one or more selected from the group consisting of: nucleoside sugar moiety modification, inter-nucleotides linkage modification, cholesterol conjugation, locked nucleic acid (LNA) modification, peptide conjugation, lipid conjugation, halogen modification, hydrocarbon group modification, and nucleic acid modification.

In another preferred embodiment, the nucleoside sugar moiety modification comprises 2'-O-methyl modification, 2'-O-methoxyethyl modification, 2'-O-alkyl modification, 2'-fluoro modification, sugar ring modification, locked nucleic acid (LNA) modification; and/or
said inter-nucleotide linkage modification comprises phosphorothioate modification alkylphosphonate modification; and/or
said nucleic acid modification comprises a "TT" modification.

In a second aspect of the present invention, provided is a short hairpin RNA (shRNA), wherein the shRNA is capable of being processed into the small RNA according to the first aspect of the present invention in a host.

In another preferred embodiment, said shRNA is multicopy.

In another preferred embodiment, said shRNA comprises a plurality of the small RNAs according to the first aspect of the present invention, wherein a plurality of small RNAs are identical or different.

In a third aspect of the present invention, provided is a polynucleotide, wherein said polynucleotide is capable of being transcribed in a host to generate the small RNA according to the first aspect of the present invention, or the short hairpin RNA (shRNA) according to the second aspect of the present invention.

In another preferred embodiment, said polynucleotide has a sequence as shown in SEQ ID NO.55.

In another preferred embodiment, said polynucleotide has a construct as shown in Formula (III):

Seq_{forward}-X-Seqᵣₑᵥₑᵣₛₑ (III),

wherein,
Seq_{forward} is the nucleotide sequence that can be processed in a host into the small RNA according to the first aspect of the present invention;
Seqᵣₑᵥₑᵣₛₑ is a nucleotide sequence substantially complementary or completely complementary to the Seq_{forward};
X is a spacer sequence between Seq_{forward} and Seqᵣₑᵥₑᵣₛₑ, and the spacer sequence is not complementary to Seq_{forward} or Seqᵣₑᵥₑᵣₛₑ;
and when the construct as shown in Formula (III), being transferred into host cells, can form a second construct as shown in Formula (IV): 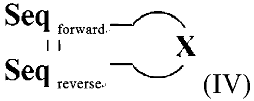
wherein, the Seq_{forward}, Seqᵣₑᵥₑᵣₛₑ and X are defined the same as above,
|| represents the base complementary pairing relation formed between Seq_{forward} and Seqᵣₑᵥₑᵣₛₑ.

In a fourth aspect of the present invention, provided is an expression vector; wherein said expression vector comprises the small RNA according to the first aspect of the present invention, or the shRNA according to the second aspect of the present invention, or the polynucleotide according to the third aspect of the present invention.

In another preferred embodiment, said expression vector comprises: viral and non-viral vectors.

In another preferred embodiment, said expression vector is a plasmid.

In a fifth aspect of the present invention, provided is a host cell; wherein said host cell comprises the expression vector according to the fourth aspect of the present invention, or has integrated into its genome the polynucleotide according to the third aspect of the present invention.

In another preferred embodiment, said host cell comprises prokaryocytes and eukaryocytes.

In another preferred embodiment, said host cell is selected from the group consisting of: *Escherichia coli,* Actinobacteria, yeast, probiotic sporeformer, *Clostridium butyricum,* Lactobacillus, Bifidobacterium, Actinomyces, filamentous fungi, and combinations thereof.

In a sixth aspect of the present invention, provided is a pharmaceutical composition; wherein said pharmaceutical composition comprises:
(a) an active ingredient; wherein said active ingredient is selected from the group consisting of: the small RNA, or an RNA or DNA complementary or reverse complementary thereto, or a modified derivative thereof according to the first aspect of the present invention; the shRNA according to the second aspect of the present invention; the expression vector according to in the fourth aspect of the present invention; or the host cell according to the fifth aspect of the present invention; and
(b) a pharmaceutically acceptable carrier.

In a seventh aspect of the present invention, provided is a veterinary pharmaceutical composition; wherein said veterinary composition comprises:
(A) a first active ingredient selected from the group consisting of:
   (A1) the small RNA, or an RNA or DNA complementary or reverse complementary thereto, or a modified derivative thereof according to the first aspect of the present invention; the shRNA according to the second aspect of the present invention; the expression vector according to the fourth aspect of the present invention; or the host cell according to the fifth aspect of the present invention;
   (A2) a small RNA, MIR-2911, which has the nucleotide sequence of SEQ ID NO.54, an RNA or DNA complementary or reverse complementary thereto, or a modified derivative thereof; a precursor RNA thereof; or a vector or host cell expressing the same; and
   (A3) a combination of (A1) and (A2) above; and
(B) a veterinary pharmaceutically acceptable carrier.

In another preferred embodiment, the dosage form of said veterinary pharmaceutical composition is an oral, injectable or topical dosage form.

In another preferred embodiment, said veterinary pharmaceutically acceptable carrier is selected from the group consisting of: water, saline, liposomes, lipids, proteins, protein-antibody conjugates, peptides, cellulose, nanogels, nanoparticles, and combinations thereof.

In another preferred embodiment, the dosage form of said veterinary pharmaceutical composition is a liquid dosage form, preferably injectables, and more preferably intravenous or intraperitoneal injectables.

In another preferred embodiment, said veterinary pharmaceutical composition further comprises a second active ingredient; wherein the second active ingredient is a medicament that is different from the first active ingredient and for the prevention, treatment and/or alleviation of FIP, or a medicament for the inhibition of FIPV, or combinations thereof.

In another preferred embodiment, said second active ingredient comprises immunosuppressants, immunomodulators, nutritional supplements, or combinations thereof.

In an eighth aspect of the present invention, provided is a veterinary feed; wherein the veterinary feed comprises:
(A) a first active ingredient selected from the group consisting of:
   (A1) the small RNA, or an RNA or DNA complementary or reverse complementary thereto, or a modified derivative thereof according to the first aspect of the present invention; the shRNA according to the second aspect of the present invention; the expression vector according to the fourth aspect of the present invention; or the host cell according to the fifth aspect of the present invention;
   (A2) a small RNA, MIR-2911, which has the nucleotide sequence of SEQ ID NO.54, an RNA or DNA complementary or reverse complementary thereto, or a modified derivative thereof; a precursor RNA thereof; or a vector or host cell expressing the same; and
   (A3) a combination of (A1) and (A2) above; and
(B) a feed acceptable carrier.

In a ninth aspect of the present invention, provided is an active ingredient for use in the preparation of a formulation or pharmaceutical composition, characterized in that, said active ingredient is selected from the group consisting of:
(A1) the small RNA, or an RNA or DNA complementary or reverse complementary thereto, or a modified derivative thereof according to the first aspect of the present invention; the shRNA according to the second aspect of the present invention; the expression vector according to the fourth aspect of the present invention; or the host cell according to the fifth aspect of the present invention;
(A2) a small RNA, MIR-2911, which has the nucleotide sequence of SEQ ID NO.54, an RNA or DNA complementary or reverse complementary thereto, or a modified derivative thereof; a precursor RNA thereof; or a vector or host cell expressing the same; and
(A3) a combination of (A1) and (A2) above;

The formulation or pharmaceutical composition is used for:
(i) preventing, treating and/or alleviating FIP; and/or
(ii) inhibiting FIPV.

In a tenth aspect of the present invention, provided is a method for non-therapeutically inhibiting FIPV *in vitro,* comprising the step of:
contacting FIPVs or FIPV-infected cells with the small RNA, or an RNA or DNA complementary or reverse complementary thereto, or a modified derivative thereof according to the first aspect of the present invention; the shRNA according to the second aspect of the present invention; the expression vector according to the fourth aspect of the present invention; the host cell according to the fifth aspect of the present invention; the pharmaceutical composition according to the sixth aspect of the present invention; or the veterinary pharmaceutical composition according to the seventh aspect of the present invention; thereby inhibiting the FIPV.

In another preferred embodiment, said cells are derived from a mammal, preferably from a cat.

In another preferred embodiment, said cells are selected from the group consisting of: renal cells, brain astrocytes, skin fibroblasts, muscle fibroblasts, lung fibroblasts, kidney fibroblasts, embryonic fibroblasts, chondrocytes, osteoblasts, skeletal muscle cells, mesenchymal stem cells, aortic endothelial cells, venous endothelial cells, macrophages, lymphoblasts, T-lymphoblasts, peripheral blood mononuclear cells, tongue cells, lung cells, and brain cells.

In an eleventh aspect of the present invention, provided is a method for preventing and/or treating FIP, comprising the step of:
administering to a subject in need the small RNA, or an RNA or DNA complementary or reverse complementary thereto, or a modified derivative thereof according to the first aspect of the present invention; the shRNA according to the second aspect of the present invention; the expression vector according to the fourth aspect of the present invention; the host cell according to the fifth aspect of the present invention; the pharmaceutical composition according to the sixth aspect of the present invention; the veterinary pharmaceutical composition according to the seventh aspect of the present invention; or the veterinary feed according to the eighth aspect of the present invention.

In another preferred embodiment, said subject is a mammal, more preferably a cat.

It is to be understood that within the scope of the present invention, the various technical features of the present invention described above and those specifically described in the following sections (such as the examples) may be combined with each other to constitute new or preferred technical solutions. For the sake of brevity, not all such combinations are exhaustively described herein.

### DESCRIPTION OF THE DRAWINGS

Figures below are to illustrate the specific embodiments of the present invention, not to limit the scope of the present invention as defined by the claims.
Fig. 1 shows the bioinformatic prediction results on the minimum free energy of part of the small RNA sequences to FIPV.
Fig. 2 shows the standard curve for the detection of small RNAs *in vivo.*
Fig. 3 shows the X-ray image of the subject before treatment in example 5.
Fig. 4 shows the X-ray image of the subject after treatment in example 5.

### DETAILED DESCRIPTION OF INVENTION

The inventor has developed for the first time through extensive and in-depth research a series of small nucleic acid for the treatment of FIP and pharmaceutical compositions comprising the same. The present invention discovers through screening and validation that, designed RNA sequences with the core sequence GCCGGG of the present invention can bind to multiple gene locus of FIPV, target directly at FIPV *in vitro,* and formulations comprising designed RNA sequences with the core sequence GCCGGG as the active ingredient can achieve anti-viral effects in animals *in vivo.* Basing on such discovery, the present invention is accomplished.

### DEFINITIONS

Unless otherwise defined, all technical and scientific terms used herein have the same meaning as commonly understood by one of ordinary skill in the art to which this invention belongs.

As used herein, the term "about," when referring to a specifically enumerated value, means that the value may vary from the enumerated value by no more than 1%. For example, as used herein, the expression "about 100" includes 99 and 101 and all values in between (e.g., 99.1, 99.2, 99.3, 99.4, etc.).

As used herein, the terms "comprising" and "including" may be open-ended, semi-closed, or closed. In other words, the terms also include "consisting substantially of" or "consisting of."

As used herein, the terms "host", "subject," and "subject in need thereof" refer to any mammal or non-mammal. Mammals include, but are not limited to, cats, other vertebrates such as rodents, humans, non-human primates, for examples, cattle, horses, dogs, pigs, sheep, goats, giraffes, deer, camels, antelopes, rats, mice, hares, and rabbits.

### Feline Infectious Peritonitis (FIP)

As used herein, the terms "Feline Infectious Peritonitis" and "FIP" refer to a severe systemic disease in cats caused by feline coronavirus infection. Feline coronavirus is a highly common pathogen, with a seroprevalence rate of 12-16% in single-cat households and 24-87% in multi-cat environments. While most infected cats exhibit only mild symptoms, approximately 5-12% of infections develop to severe clinical symptoms, which can lead to the fatal systemic condition known as Feline Infectious Peritonitis.

As used herein, the terms "Feline Infectious Peritonitis Virus" and "FIPV" refer to the virus responsible for causing FIP. FIPV belongs to the genus *Coronavirus* within the family *Coronaviridae.* Its genome consists of a non-segmented, single-stranded, positive-sense RNA molecule, approximately 30 kb in size. The virus can replicate in various feline cell types and is also capable of infecting suckling mice, rats, and hamsters. Transmission can occur via insect vectors, the fecal-oral route, and vertically.

### RNAi and anti-virus infection

RNAi (RNA interference) is a specific gene silencing phenomenon mediated by double or single-stranded small RNAs and involving the participation of specific enzymes. Both double-stranded small RNAs (siRNAs) and single-stranded small RNAs can lead to mRNA degradation and/or translational inhibition through specific binding to target mRNA, thereby blocking gene expression at the transcriptional, post-transcriptional, and translational levels. The RNAi phenomenon was first discovered in 1990 during research on transgenic plants. In 2001, scientists successfully induced RNAi in cultured mammalian cells, suggesting the potential of using RNAi technology to treat viral infectious diseases. In recent years, with progress in RNAi research, more effective approaches for the treatment of viral diseases have been provided.

### siRNA and short hairpin RNA (shRNA)

SiRNAs are a class of double-stranded RNA molecules, approximately 21-25nt in length. They are processed from endogenous or exogenous long double-stranded RNA precursors by Dicer (an RNase III family enzyme specific for double-stranded RNA). siRNAs can bind to target mRNA through complete complementarity, leading to its degradation and consequent silencing of the target gene. siRNA was first discovered by the David Baulcombe team in the UK as part of the post-transcriptional gene silencing (PTGS) phenomenon in plants. In 2001, the team led by Thomas Tuschl discovered that synthetic siRNAs, complementary to specific gene sequences in mammals, could induce degradation of the corresponding messenger RNA (mRNA), thereby preventing its translation into protein, i.e., producing an RNAi effect. This finding triggered the use of controllable RNAi methods in biomedical research and drug development.

Short hairpin RNA (shRNA) consists of both sense and antisense strands separated by a loop sequence, and is commonly used in the construct of expression vectors. shRNA is synthesized in the nucleus of transfected/transduced cells and is processed into siRNA in the cytoplasm via the same processing mechanism as miRNA.

The present invention provides a class of small RNAs that inhibit FIPV. These small RNAs can be siRNAs that can be produced by shRNA processing.

### MicroRNA (miRNA) and its precursor

MicroRNA (miRNA) is a class of endogenous, non-coding, single-stranded small RNAs, approximately 22 nucleotides in length, discovered in recent years in eukaryotes such as nematodes, fruit flies, plants, and mammals. Their expression exhibits tissue and temporal specificity. By base-pairing with target mRNAs, miRNAs negatively regulate gene expression at the post-transcriptional level, leading to mRNA degradation or translational inhibition, rendering them crucial regulatory molecules that modulate the expression of other functional genes. Growing evidence indicates that despite of their small size, miRNAs play vital roles in various biological processes by forming complete or incomplete complementary pairs with target mRNAs.

MicroRNAs are a class of endogenous, non-coding, single-stranded RNA molecules that regulate gene transcription and translation *in vivo.* The seed sequence (nucleotides 2-8) from the 5' end is highly evolutionarily conserved and serves as a key element for post-transcriptional gene regulation in the cytoplasm (Bioinformatics. 2014;30(10):1377-83). Besides the canonical function of the seed region, miRNAs possess numerous other regulatory mechanisms and functions that are not yet fully understood. For instance, microRNA-552-3p can not only exert the canonical post-transcriptional inhibition on the target gene of cytochrome P450 enzyme CYP2E1 via seed sequence binding to the mRNA 3'-UTR, but non-canonical inhibition at the transcriptional level by complementary binding of its non-seed sequence to the gene's promoter region (Biochim Biophys Acta. 2016;1859(4):650-62). This suggests that miRNA-mediated regulation of target genes may not rely solely on the seed sequence but may also depend on sequences outside the seed region.

The present invention provides a class of small RNAs that inhibit FIPV. These small RNAs may resemble microRNAs in their structure, function, and metabolic pathways, and may also be referred to as artificial microRNAs or microRNA analogs. As used herein, the term "microRNA" refers to a class of RNA molecules processed from transcripts that can form miRNA precursors. Mature miRNAs typically range from 18 to 26 nucleotides (nt) in length (more specifically, about 19-23nt), without excluding miRNA molecules of other lengths. In a preferred embodiment of the present invention, mature miRNAs are 18-30 nucleotides in length. miRNAs are typically detectable by Northern blotting.

MicroRNAs can be isolated from cells. As used herein, "isolated" means that the substance has been separated from its original environment (for a naturally occurring substance, the original environment is the natural environment). For example, polynucleotides and polypeptides in their natural state within living cells are not isolated or purified. However, the same polynucleotides or polypeptides are considered isolated and purified when being separated from other components co-existing in their natural state.

MicroRNAs can be processed from precursor miRNAs (pre-miRNAs), which can fold into a stable stem-loop (hairpin) structure, typically 50-100bp in length. The stem-loop structure comprises two flanking sequences in the stem that are substantially complementary. The precursor miRNA can be naturally occurring or artificially synthesized.

A precursor miRNA can be cleaved to generate a miRNA. The miRNA can be substantially complementary to at least part of the mRNA of a coding gene. As used herein, "substantially complementary" means that the nucleotide sequences are complementary enough to interact in a predictable manner, such as by forming a secondary structure (e.g., a stem-loop structure). Generally, two "substantially complementary" nucleotide sequences share at least 70% nucleotide complementarity; preferably, at least 80% nucleotide complementarity; more preferably, at least 90% nucleotide complementarity; even more preferably, at least 95% nucleotide complementarity; such as 98%, 99%, or 100%. Generally, two sufficiently complementary molecules can have up to 5 mismatched nucleotides, e.g., 1, 2, 3, 4, or 5 mismatched nucleotides.

As used in this application, a "hairpin" structure, also known as a "stem-loop" structure, refers to a nucleotide molecule that can form a secondary structure comprising a double-stranded region (the stem) formed by two regions of the nucleotide molecule (located on the same molecule) flanking the double-stranded portion; it also includes at least one "loop" structure comprising non-complementary nucleotides, i.e., a single-stranded region. The double-stranded portion of the nucleotide molecule can remain double-stranded even if the two regions are not perfectly complementary. For example, insertions, deletions, or substitutions may cause a small region to be non-complementary or to form its own stem-loop or other secondary structure; nevertheless, the two regions can still be substantially complementary and interact in a predictable manner to form the double-stranded region of the stem-loop structure. Stem-loop structures are well-known to those skilled in the art. Typically, upon obtaining a nucleic acid with a primary nucleotide sequence, a person skilled in the art can determine whether it can form a stem-loop structure.

The small RNA according to the present invention refers to a small RNA with the core sequence GCCGGG and a length of 18-30nt.

In an embodiment, the small RNA of the present invention has a sequence construct from the 5' to the 3' end as shown in Formula (I) below:

(P)n-R-(Q)m (I);

wherein,
P is selected from A, U, G or C;
R is the core sequence, which has the nucleotide sequence GCCGGG;
Q is selected from A, U, G or C;
and, (P)n represents n number of P, with n being an integer selected from 0 to 4,
(Q)m represents m number of Q, with m being an integer selected from 9 to 24.

In an embodiment of the present invention, the RNA of the present invention has a nucleotide sequence selected from the group consisting of SEQ ID NOs. 1-53.

The medicament of the present invention comprises the designed single or double-stranded RNA comprising the core sequence, or a DNA that is completely or partially complementary to the single or double-stranded RNA, and pharmaceutically acceptable carriers as well as pharmaceutically acceptable excipients.

In the present invention, appropriate modification can also be made to the RNA to enhance pharmaceutical stability or improve its targeting specificity to particular organs. A person of ordinary skill in the art may employ conventional methods to modify the small RNA of the present invention. Modification approaches include, but are not limited to: methylation, hydrocarbon group modification, glycosylation (e.g., 2'-O-methyl sugar moiety modification, alkyl-sugar modification, sugar ring modification, etc.), nucleotide addition, peptide conjugation, lipid conjugation, halogen modification, and nucleic acid modification (e.g., "TT" modification), and etc.

In one embodiment, the modified small RNA derivative is a compound monomer having the structure as shown in Formula (II), or a multimer thereof:

(X)r-(Y)s (II)

wherein,
each X is a small RNA according to the first aspect of the present invention;
each Y is independently a modifier that promotes the stability of the small RNA drug administration;
Y is conjugated at the left, right side or in the middle of X;
r is a positive integer from 1 to 100 (preferably from 1 to 20) (preferably, r is 1, 2, 3, 4 or 5);
s is a positive integer from 1 to 1000 (preferably from 1-200);
each "-" represents a linker, a chemical bond or a covalent bond.

In one embodiment, the linker is a nucleotide sequence with a length of 1-10nt.

In one embodiment, the Y includes (but is not limited to) cholesterol, steroids, sterols, alcohols, organic acids, fatty acids, esters, monosaccharides, polysaccharides, amino acids, polypeptides, mononucleotides, or polynucleotides.

### Polynucleotide construct

Polynucleotide constructs can be designed based on the small RNA sequences provided in the present invention After introduction into cells, these constructs can be processed into small RNAs that influence the expression of corresponding mRNA; that is, said polynucleotide constructs are capable of upregulating the level of the corresponding small RNAs *in vivo.* Accordingly, the present invention provides an isolated polynucleotide (construct) that can be transcribed in human cells into a shRNA, wherein said shRNA can be cleaved and expressed as said small RNA by the cell.

As a preferred embodiment of the present invention, the polynucleotide construct comprises a structure as shown in Formula (III) below:

Seq_{forward}-X-Seqᵣₑᵥₑᵣₛₑ (III),

wherein,
Seq_{forward} is the nucleotide sequence that can be processed in cells into said small RNA, and Seqᵣₑᵥₑᵣₛₑ is the nucleotide sequence substantially complementary to the Seq_{forward}; or, Seqᵣₑᵥₑᵣₛₑ is the nucleotide sequence that can be processed in cells into said small RNA, and Seq_{forward} is the nucleotide sequence substantially complementary to the Seqᵣₑᵥₑᵣₛₑ; X is a spacer sequence between Seq_{forward} and Seqᵣₑᵥₑᵣₛₑ, and the spacer sequence is not complementary to Seq_{forward} or Seqᵣₑᵥₑᵣₛₑ;

Being introduced into cells, the construct as shown in Formula (III) forms a secondary construct as shown in Formula (IV): 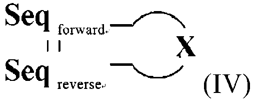
wherein, the Seq_{forward}, Seqᵣₑᵥₑᵣₛₑ and X are defined the same as above,
∥ represents the base complementary pairing relation formed between Seq_{forward} and Seqᵣₑᵥₑᵣₛₑ.

Typically, said polynucleotide construct is comprised in an expression vector. Accordingly, the present invention further comprises a vector comprising said small RNA or said polynucleotide construct. The expression vector typically further comprises elements such as a promoter, an origin of replication, and/or a marker gene. Those skilled in the art can construct the expression vectors required for the present invention using their well-known methods, including *in vitro* recombinant DNA techniques, DNA synthesis techniques, *in vivo* recombination techniques, and the like. Preferably, the expression vector comprises one or more selectable marker genes to provide phenotypic traits for the selection of transformed host cells, such as kanamycin, gentamicin, hygromycin, or ampicillin resistance.

In the present invention, the expression vector is not particularly limited and includes commercially available vectors or those prepared by conventional methods. Representative examples include, but are not limited to: pcDNATM6.2-GW/miR, pcDNA3, pMIR-REPORT miRNA, pAdTrack-CMV, pCAMBIA3101+pUC-35S, pCMVp-NEO-BAN, pBI121, pBin438, pCAMBIA1301, pSV2, CMV4 expression vector, pmiR-RB-Report^{™}, pshOK-basic, mmu-mir 300 - 399 miRNASelect^{™}, pshRNA-copGFP Lentivector, GV317, GV309, GV253, GV250, GV249, GV234, GV233, GV232, GV201, GV159 or other GV series expression vectors.

In an embodiment of the present invention, plasmids, AAV vectors or combinations thereof can be used.

In another preferred embodiment, in said expression vector, the promoter operably linked to the polynucleotide encoding the precursor RNA comprises a constitutive promoter or a tissue-specific promoter, preferably a promoter that initiates transcription specifically in liver tissue. In other words, these promoters are used to drive the expression of the precursor RNA.

Representative promoters include, but are not limited to: pCMV promoter, U6, H1, CD43 promoter, CD45 (LCA) promoter, CD68 promoter, Endoglin (CD105) promoter, Fibronectin promoter, Flt-1 (VEGFR-1) promoter, GFAP promoter, GPIIb (Integrin αIIb) promoter, ICAM-2 (CD102) promoter, MB (Myoglobin) promoter, NphsI (Nephrin) promoter, SPB promoter, SV40 / hAlb promoter, SYN1 promoter, WASP promoter, and combinations thereof.

### Pharmaceutical composition and administration method thereof

The present invention provides a pharmaceutical composition, wherein said pharmaceutical composition comprises:
(a) an active ingredient; the active ingredient is selected from the group consisting of: the small RNA, an RNA or DNA complementary or reverse complementary thereto, or a modified derivative thereof according to the first aspect of the present invention; the shRNA according to the second aspect; the expression vector according to the fourth aspect; and the host cell according to the fifth aspect of the present invention;
(b) a pharmaceutically or veterinary pharmaceutically acceptable carrier.

The pharmaceutical composition of the present invention comprises the designed single or double-stranded RNA comprising the core sequence, or a DNA fully or partially complementary thereto, and a pharmaceutically (or veterinary pharmaceutically) acceptable carrier, and optionally, pharmaceutically (or veterinary pharmaceutically) acceptable excipients.

As used herein, the terms "effective amount" or "effective dose" refer to a quantity capable of producing a functional or active effect in humans and/or animals, which is acceptable to humans and/or animals.

As used herein, the term "pharmaceutically (or veterinary pharmaceutically) acceptable" refers to a component suitable for use in felines and/or mammals without excessive adverse side effects (such as toxicity, irritation, and allergic reactions), i.e., a component possessing a reasonable benefit/risk ratio. The term "pharmaceutically (or veterinary pharmaceutically) acceptable carrier" refers to a carrier for therapeutic agent administration, including various excipients and diluents.

The pharmaceutical composition of the present invention comprises a safe and effective amount of the active ingredient of the present invention and a pharmaceutically (or veterinary pharmaceutically) acceptable carrier. Such carriers include, but are not limited to: saline, buffers, glucose, water, glycerol, ethanol, and combinations thereof. The pharmaceutical formulation should generally be compatible with the intended mode of administration. The dosage forms of the composition include injectables, oral preparations (tablets, capsules, oral solutions), transdermal agents, and sustained-release formulations. For example, preparation can be carried out by conventional methods using physiological saline or aqueous solutions containing glucose and other adjuvants. The pharmaceutical composition is preferably manufactured under sterile conditions.

The effective amount of the active ingredient of the present invention can vary depending on factors such as the administration mode and the severity of the disease to be treated, and etc. Selection of a preferred effective amount can be determined by a person skilled in the art based on various factors (e.g., through clinical trials). These factors include, but are not limited to: pharmacokinetic parameters of the active ingredient such as bioavailability, metabolism, half-life, etc.; severity of the disease to be treated in the patient; the patient's body weight; the patient's immune status; and the route of administration, etc. Typically, satisfactory effects can be obtained when the active ingredient of the present invention is administered daily at a dose of about 0.00001 mg to 50 mg per kg of animal body weight (preferably 0.1 mg to 15 mg per kg of animal body weight). For example, depending on the exigencies of the therapeutic situation, several divided doses may be administered daily, or the dose may be proportionally reduced.

Pharmaceutically (or veterinary pharmaceutically) acceptable carriers in the present invention include, but are not limited to: water, saline, liposomes, lipids, proteins, protein-antibody conjugates, peptides, cellulose, nanogels, nanoparticles, or combinations thereof. The selection of the carrier should be compatible with the mode of administration, all of which are well-known to those of ordinary skill in the art.

In the present invention, the expression vector may be administered directly to a subject, or may be formulated with a pharmaceutically (or veterinary pharmaceutically) acceptable carrier into a pharmaceutical composition for administration. Said administration includes intravenous injection.

### Major advantages of the present invention include:

1) The small RNA of the present invention features fairly strong binding ability to FIPV gene, especially to the S gene related to the infectivity and systematic toxicity of FIPV, thus blocking the infection and reducing the toxicity of the virus. Meanwhile, the small RNA can bind to genes related to the replication function of FIPV genome, thus blocking the virus replication and transmission.
2) The small RNA of the present invention can inhibit FIPV in clinical cases, alleviating the symptoms of animals.

The present invention is further illustrated by the following specific examples. It is to be understood that these examples are provided solely for the purpose of illustrating the invention and are not to be construed as limiting the scope of the invention. Experimental procedures in the following examples without specific conditions are generally conducted under conventional conditions, such as those described in Sambrook et al., Molecular Cloning: A Laboratory Manual (New York: Cold Spring Harbor Laboratory Press, 1989), or according to the manufacturer's suggested conditions. Unless otherwise indicated, all percentages and parts are by weight.

### I. Bioinformatic prediction

It can be known by inquiring gene databases that, FIPV genome, with a size of 29.4 kb, is reported of containing 10 genes, namely, S (spike protein), nsp 7a, nsp 3b, ORF 1a/1b, nsp 3c, nsp 7b, N, M, E and nsp 3a. The binding sites with MFE (Minimal Free Energy) lower than -15.0 kcal/mol of Populus euphratica-derived peu-MIR2911 to FIPV were predicted using RNAhybrid software (Version: RNAhybrid 2.2), with the 2-8nt seed region set as a necessary matching condition. 32 matching binding sites were obtained, which distribute across almost all genes, and among which 19 sites are with MFE less than -20.0kcal/mol. The peu-MIR2911 has 5 binding sites to the S gene that is related to the infectiousness and systematic toxicity, 3 out of which are with MFE less than -20kcal/mol (2808, 3188, 3682), indicating that MIR2911 is capable of blocking virus infection and replication, and reducing toxicity; 19 binding sites to the ORF 1a/1b that is related to the replication function of the FIPV genome, 11 out of which are with MFE less than -20kcal/mol, indicating that MIR2911 is capable of blocking virus replication and transmission.
sequence of peu-MIR2911 (5'-3'):
CGGCCGGGGGACGGGCUGGG(SEQ ID NO.54)

### II. Sequence design and selection

Taking the 3-8nt of peu-MIR2911 as the core sequence, small RNA sequences are designed and screened.
Core sequence: GCCGGG

Design rule of said active ingredient small RNA sequence: random 0-4nt can be added at the beginning of the sequence, and random 9-24nt can be added at the end of the sequence, so that the total length of the sequence is 18-30nt.

53 RNA sequences were designed and selected basing on the rule above.

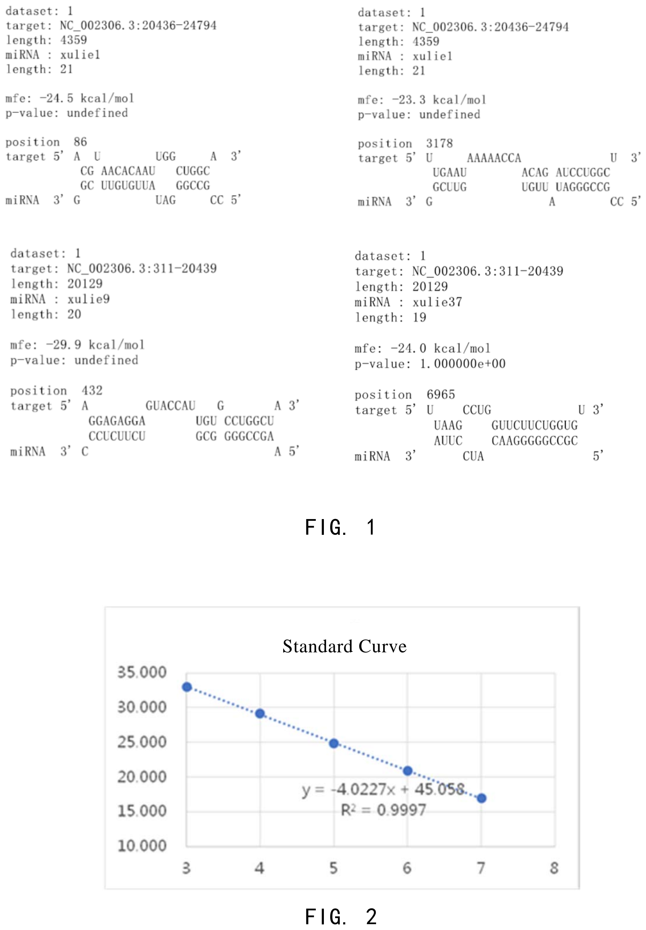

The binding status of the selected RNA sequences with FIPV genome is shown as Table 1 below. The binding condition of some RNAs to binding sites is shown in Fig. 1. Cellular assay methods are asshown in Example 1.

**Table 1 RNA sequences of the present invention**

| N O. | Sequence (5'-3') | mfe, kcal/mol | Target gene or locus | Cell tested activity |
|---|---|---|---|---|
| 1 | CCGCCGGGAUAUUGUGUUCGG | -24.5 | S | + |
| 2 | UAGGCCGGGGGACUGUACUGAUGUU | -22.9 | M | + |
| 3 | CCAGCCGGGGGAGACAUCCUCGGG | -18.9 | S | + |
| 4 | AUCGGCCGGGGGUUCCCUAUCGGCA | -29.5 | ORF1a\1b | + |
| 5 | CAGGCCGGGGGAUGCUCGCAAAGCU | -29.2 | ORF1a\lb | + |
| 6 | GCAGCCGGGGGCGAACAUCGCACG | -25.6 | ORF1a\1b | + |
| 7 | AUGCCGGGGGCGACAAAGAAUCUUUU | -32.4 | ORF1a\1b | + |
| 8 | ACGCCGGGGGAUAAAUCCACGCCUC | -27.6 | ORF1a\lb | + |
| 9 | AAGCCGGGGCGUCUUCUCCC | -29.9 | ORF1a\1b | + |
| 10 | UUGGCCGGGGGCCUCCGCGCGG | -33.6 | ORF1a\lb | + |
| 11 | UAGGCCGGGGGCUGGUCUGGGUUUAU | -29.5 | ORF1a\1b | + |
| 12 | GUGGCCGGGGGCGAGUAUCCCUCCGC | -36.7 | ORF1a\lb | + |
| 13 | CGCGCCGGGGGCCAGCUUAAUCUCC | -33.6 | ORF1a\lb | + |
| 14 | UCGGCCGGGGGCGAGUGACCGCAAUU | -32.9 | ORF1a\1b | + |
| 15 | ACGCCGGGGGAUAUUCCGAACCUGC | -26.9 | ORF1a\lb | + |
| 16 | GCUGCCGGGGGGGUGACUGUGCAUA | -36.8 | ORF1a\lb | + |
| 17 | CCAGCCGGGGGCGCCCCGGUCGAU | -26.5 | ORF1a\1b | + |
| 18 | CGGCCGGGGGCUCCACCGGAGAUA | -30.9 | ORF1a\lb | + |
| 19 | GGCCGGGGGACUUUCGGAGAGCGUGA | -32.2 | ORF1a\lb | + |
| 20 | GGGCCGGGGUAUGUUUUCGG | -30.4 | ORF1a\1b | + |
| 21 | UGGCCGGGGGCUGCCCCAAGACUG | -26.9 | ORF1a\lb | + |
| 22 | AGCCGGGGGGAGGGUCUGCAAG | -27.5 | ORF1a\lb | + |
| 23 | CUAGCCGGGGGAGCUGGUCAUACUC | -27.6 | ORF1a\lb | + |
| 24 | CUCCGCCGGGGGAAUCCCUGCGGAUA | -28.7 | ORF1a\lb | + |
| 25 | UGGCCGGGGGAGUUGAUUAAUA | -25.3 | ORF1a\1b | + |
| 26 | CAGCCGGGGGCAGCUUAGGCGACA | -27.9 | ORF1a\lb | + |
| 27 | UCGCCGGGGGUAUCUAACGUGUUC | -31.8 | ORF1a\lb | + |
| 28 | UGGCCGGGGGGGGGGUCGGUC | -30.7 | ORF1a\lb | + |
| 29 | ACCGCCGGGGGCAAUACAAAGAG | -24.6 | ORF1a\lb | + |
| 30 | UUGGCCGGGGGAGGCCUAGUUGGAAU | -35.7 | ORF1a\1b | + |
| 31 | GCGCCGGGGGUUCGACAGGGAAAAGA | -27.4 | ORF1a\lb | + |
| 32 | AUGCCGGGGGGCAGUUAGAAGCGAU | -29.6 | ORF1a\lb | + |
| 33 | UGGCCGGGGGAUACGUGCGUA | -26.8 | ORF1a\lb | + |
| 34 | GGGCCGGGGGCAUAGUACUCUCCCG | -35.8 | ORF1a\lb | + |
| 35 | CCGCCGGGGGAUAAAGAGCAAA | -23.6 | ORF1a\lb | + |
| 36 | GCCGGGGGCCUCAAAUCCUCGCCA | -30.1 | ORF1a\lb | + |
| 37 | CGCCGGGGGAACAUCCUUA | -29.9 | ORF1a\lb | + |
| 38 | CGCCGGGGGCCCGGCUCUUCC | -30.5 | ORF1a\lb | + |
| 39 | UCGCCGGGGGUUACUUCAGAUUA | -27.6 | ORF1a\1b | + |
| 40 | UUAGCCGGGAAUUCAGCACGACCAA | -27.5 | ORF1a\lb | + |
| 41 | AUGCCGGGGGCUGUAUACCGAU | -30.4 | ORF1a\1b | + |
| 42 | AAGGCCGGGGGGAAGGAAACGCUCUA | -25.1 | ORF1a\1b | + |
| 43 | GUGCCGGGGGGGUACGUCACGACUAU | -29.5 | ORF1a\1b | + |
| 44 | ACGCCGGGGGCGUGGUACUACCAU | -26 | ORF1a\1b | + |
| 45 | GAUGCCGGGGGACGUUGCUGUUUCC | -31.4 | ORF1a\1b | + |
| 46 | GAGCCGGGGGUAUGGCGGCGAUG | -30.8 | ORF1a\1b | + |
| 47 | GAGCCGGGGGGAUCAACUAGGUUU | -27.8 | ORF1a\1b | + |
| 48 | UGCCGGGGGCGGUCUAUUUCGGGA | -30.1 | ORFla\lb | + |
| 49 | CAGCCGGGGGCUCAGGCCAUCCCC | -27.2 | ORF1a\1b | + |
| 50 | GGCCGGGGGCCAGGCGUCCCCCC | -28.2 | ORFla\lb | + |
| 51 | UUGCCGGGGGAAGACUCCUAUCCG | -27.3 | ORFla\lb | + |
| 52 | AUGGCCGGGGGGAGGGAUGUCUUACU C | -26.8 | ORF1a\1b | + |
| 53 | CGGCCGGGGGGUCAAAGCUUCGUGU | -28.4 | ORFla\lb | + |
| 54 | CGGCCGGGGGACGGGCUGGG | -27.1 | ORF1a\1b | + |

### Example 1. SEQ ID NO.1 expressing-plasmids can significantly inhibit FIPV in vitro

CRFK (Crandell-Rees Feline Kidney) cells were inoculated at 1 × 10⁵ /mL on 96-well plate in DMEM medium (2.0mM L-glutamine, 100 units/ml penicillin and 100 µg/ml streptomycin) added with 10% fetal bovine serum (FBS), cultivated for 48 h at 37°C, 5% CO₂, and then used for experiment after passaging 3 times at 1:3.

FIPV-1146 (feline infectious peritonitis virus) was bought from ATCC. Before use, pre-titrated virus aliquots were removed under -70°C condition and thawed to room temperature in a biosafety cabinet.

CRFK cells for experiment were divided into two groups, of which one is the negative control, and the other was added with 0.01 MOI feline corona virus (FIPV) to each well. After infection for 1 day, cell viability was detected using CCK-8 assay, and difference in cell viability between the uninfected and infected samples were compared.

Then the experiment group cells were co-incubated with the plasmids carrying the SEQ ID NO.1 expressing-DNA sequence (CCGCCGGGATATTGTGTTCGG, SEQ ID NO. 55) (plasmid: Lipo3000 = 1:3, 5 ng plasmids), and the control plasmid group cells were co-incubated with plasmids carrying a non-functional sequence. Differences in cell viability were detected on day 2 and day 3, and the results are shown in the table below:

**Table 2 Effect of plasmids of the present invention on the viability of FIPV infected cells**

| Group | Before plasmid transfection | After plasmid transfection | |
|---|---|---|---|
| | CCK-8 | CCK-8: 24h | CCK-8: 48h |
| Blank group (cell medium) | 0.3791 | 0.3756 | 0.3799 |
| Negative control group (CRFK + no treatment) | 1.1261 | 1.0389 | 0.9925 |
| Virus control group (CRFK + FIPV) | 0.7672 | 0.5108 | 0.4621 |
| Plasmid control group (CRFK + FIPV + plasmid carrying a non-functional sequence) | | 0.4613 | 0.4866 |
| Experiment group (CRFK + FIPV + plasmid carrying a treatment functional sequence CCGCCGGGATATTGTGTTCGG) | | 0.8737 | 0.8799 |

As shown in the detection results, FIPV infection (the positive control group) has significantly inhibited the CRFK cell viability, and plasmids carrying a treatment functional sequence can effectively increase cell viability, compared with the plasmid control group.

### Example 2. Detection of expression in animals in vivo

In this example, engineered *Escherichia coli* bacteria expressing SEQ ID NO.9 were prepared using genetic recombination techniques; the product was then amplified using the fermentation method, extracted using the lysis method, and then injections meeting veterinary drug standards were obtained through purification and filling procedures.

6 nude mice (3 males, 3 females) aged 6-8W were intravenously injected with the injections at 0.2mg/kg, 1mg/kg and 5mg/kg, respectively. Blood sample was collected at 2h and 6h post-injection, and plasma was prepared. Total RNA of plasma was isolated using Trizol method, and then the small RNA expression of SEQ ID NO.9 in the total RNA was detected using PCR. The standard curve is shown as in Fig. 2. The primers and probes of PCR were commercially customized (Applied Biosciences). Before administration, the drug doses were calculated basing on the average CT value of each group mice.

The reverse transcription system and qPCR system are shown as below:
Reverse transcription system (10µl in total):

| | |
|---|---|
| dNTPs | 1µl |
| RNA Templates (1/1000) | 2µl |
| HS-1R (1/500) | 1µl |
| M-mlv | 0.3µl |
| (5 × Buffer) | 2µl |
| RNase Free Water | 3.7µl |

qPCR system (20µl in total):

| | |
|---|---|
| 2×SYBRGreenMaster Mix | 10µl |
| Forward Primer (HS-1F1/500) | 1µl |
| Reverse Primer (UR1/500) | 1µl |
| DNA Template | 2µl |
| RNase Free Water | 6µl |

The reaction program includes:
Reverse transcription reaction program:
   65°C 5min→4°C 2min→4°C+-∞→42°C 60min→70°C 10min→4°C+∞
qPCR reaction program:
   95°C 5min-40 cycles (95°C 10s→60°C 30s-72°C 20s)→95°C 15s→60 °C 60s→95°C 30s

Detection results in Table 3 show that, a high expression level of the small RNA SEQ ID NO.9 was observed in mouse blood as early as 3 hours after administration and remained elevated at 6 hours. Besides, the expression of SEQ ID NO.9 small RNA exhibits a dose-dependent response, with a detectable effect even at the low dose of 0.2 mg/kg.

**Table 3 Expression in vivo of the small RNA of the present invention**

| | **Mean CT/0.2mg/kg** | | |
|---|---|---|---|
| Mouse | 0h (before treatment) | 3h | 6h |
| Male 1 | 33.22 | 32.29 | 33.2 |
| Male 2 | 34.08 | 32.63 | 32.8 |
| Male 3 | 33.72 | 32.01 | 31.92 |
| Female 1 | 32.72 | 30.74 | 32.87 |
| Female 2 | 35.03 | 34.61 | 34.06 |
| Female 3 | 33 | 33.63 | 33.86 |

| | **Mean CT/1mg/kg** | | |
|---|---|---|---|
| Mouse | 0h (before treatment) | 3h | 6h |
| Male 1 | 33.22 | 31.95 | 31.98 |
| Male 2 | 34.08 | 32.37 | 32.29 |
| Male 3 | 33.72 | 31.66 | 29.13 |
| Female 1 | 32.72 | 30.38 | 32.01 |
| Female 2 | 35.03 | 33.71 | 31.74 |
| Female 3 | 33 | 31.63 | 32.61 |

| | **Mean CT/5mg/kg** | | |
|---|---|---|---|
| Mouse | 0h (before treatment) | 3h | 6h |
| Male 1 | 33.22 | 31.11 | 32.88 |
| Male 2 | 34.08 | 31.86 | 31.47 |
| Male 3 | 33.72 | 30.18 | 31.6 |
| Female 1 | 32.72 | 30.34 | 31.99 |
| Female 2 | 35.03 | 32.13 | 32.76 |
| Female 3 | 33 | 32.68 | 33.21 |

### Example 3 FIP Clinical case 1

A female Chinese garden cat, 3-month-old, weighing 0.9 kg. The cat presented vomiting symptoms two days ago, with an average frequency of 3-4 times per day. The vomitus consisted of yellow, foamy, viscous material. The cat had normal fecal output but exhibited anorexia while remaining mentally alert. Its vaccination history indicated only a single dose. A complete blood count (CBC) revealed elevated levels of white blood cells (leukocytes) and granulocytes, suggesting inflammation in the body.

**Table 4 Lab test results before treatment**

| **Test** | **Result** | **Reference range** | **Below normal** | **Normal** | **Above normal** |
|---|---|---|---|---|---|
| VetAutoread (1:39 pm, September 9^{th}, 2022) | | | | | |
| Hematocrit Value (HCT) | 31.8% | 24.0-45.0 | | | |
| Hemoglobin (HGB) | 10.0g/dL | 8.0-15.0 | | | |
| Mean Corpuscular Hemoglobin Concentration (MCHC) | 31.4g/dL | 30.0-36.9 | | | |
| Leukocytes | 35.20 K/µL | 5.00-18.90 | | | Above normal |
| Granulocytes (GRANS) | 26.00K/µL | 2.50-12.50 | | | Above normal |
| (%GRANS) Percentage of Granulocytes (%GRANS) | 88.5% | | | | |
| Lymphocyte-to-Monocyte Ratio (L/M) | 5.2x10^9/L | 1.5-7.8 | | | |
| Lymphocyte and Monocyte Percentage (%L/M) | 12% | | | | |
| Platelet (PLT) | 388 K/µL | 175-500 | | | |

The fecal FPV antigen test showed strong positive (FPV > 50.00); also referring to clinical symptoms, the diagnosis of FIP was confirmed.

Drug was prepared according to the method in example 2, with only one difference in sequence (SEQ ID NO.1 was adopted), and then injected intravenously at 4 mg/kg for 3 consecutive days, followed by 1 day break. A repeat fecal FPV antigen test conducted thereafter showed a value of 12.08; the symptoms of the cat were obviously improved.

### Example 4 FIP Clinical case 2

A blue cat, female, weighing 4.4 kg. Its abdominal girth had progressively increased over the past month, which was initially attributed to weight gain and thus not a major concern. Recently, deterioration in mental status and reduced activity were noted, along with a mild decrease in appetite. Physical examination revealed significantly distended abdominal girth, palpable fluid wave upon abdominal palpation without signs of pain, marked breathing difficulty, and pronounced abdominal contraction along with respiration. Fecal and ascitic fluid samples were collected, the PCR detection of which confirmed FIPV-positive status.

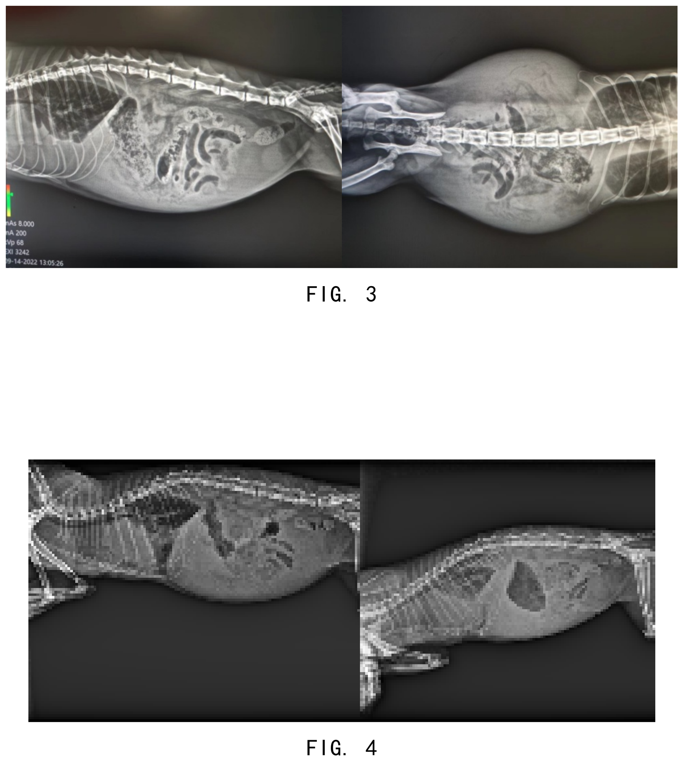

Its X-ray images are as shown in Fig. 3, in which it was clearly revealed the presence of abdominal fluid and pleural effusion which caused severe pulmonary compression.

Drug was prepared according to the method in example 2, with only one difference in sequence (SEQ ID NO.37 was adopted), and then injected intravenously at 1 mg/kg once every other day for a total of 4 times. After treatment, the pulmonary effusion was obviously reduced, and ascitic fluid condition also relieved, as shown in the X-ray image in Fig. 4.

All publications mentioned in the present invention are incorporated in the present application by reference as if each individual publication were individually incorporated by reference. Furthermore, it should be understood that after reading the foregoing teachings of the present invention, those skilled in the art may make various alterations or modifications to the invention, all of which are equivalent forms that likewise fall within the scope defined by the appended claims of the present application.

## Claims

1. A small RNA, an RNA or DNA complementary or reverse-complementary thereto, or a modified derivative thereof, wherein the small RNA comprises the core sequence GCCGGG, and the length of said small RNA is 18-30nt;
provided that the sequence of said small RNA is not as shown in SEQ ID NO.54.

2. The small RNA according to claim 1, wherein, the small RNA has a sequence construct from the 5' to the 3' end as shown in Formula (I) below:
(P)ₙ-R-(Q)ₘ (I);
wherein,
P is selected from A, U, G or C;
R is the core sequence, which has the nucleotide sequence GCCGGG;
Q is selected from A, U, G or C;
and, said (P)n represents n number of P, with n being an integer selected from 0 to 4,
said (Q)m represents m number of Q, with m being an integer selected from 9 to 24.

3. The small RNA according to claim 1, wherein, the nucleotide sequence of said small RNA is any one selected from SEQ ID NOs. 1-53.

4. The small RNA according to claim 1, wherein, the nucleotide sequence of said small RNA is selected from SEQ ID NO.1, SEQ ID NO.9 or SEQ ID NO.37.

5. A short hairpin RNA, wherein, the short hairpin RNA can be processed in host into the small RNA according to claim 1.

6. A polynucleotide, wherein, the polynucleotide can be transcribed by host into the small RNA according to claim 1, or the short hairpin RNA according to claim 5.

7. An expression vector, and said expression vector comprises the small RNA according to claim 1, or the short hairpin RNA according to claim 4, or the polynucleotide according to claim 6.

8. A host cell, wherein said host cell comprises the expression vector according to claim 7, or its genome is integrated with the polynucleotide according to claim 6.

9. A veterinary pharmaceutical composition, wherein the veterinary pharmaceutical composition comprises:
(A) a first active ingredient selected from the group consisting of:
(A1) the small RNA, an RNA or DNA complementary or reverse complementary thereto, or a modified derivative thereof according to claim 1; the short hairpin RNA according to claim 5; the expression vector according to claim 6; or the host cell according to claim 8;
(A2) a small RNA, MIR-2911, which has the nucleotide sequence of SEQ ID NO.54, an RNA or DNA complementary or reverse complementary thereto, or a modified derivative thereof; a precursor RNA thereof; or a vector or host cell expressing the same; and
(A3) a combination of (A1) and (A2) above; and
(B) a veterinary pharmaceutically acceptable carrier.

10. The veterinary pharmaceutical composition according to claim 9, wherein the veterinary pharmaceutically acceptable carrier is selected from the group consisting of: water, saline, liposomes, lipids, proteins, protein-antibody conjugates, cellulose, nanogels, nanoparticles, and combinations thereof.

11. Use of an active ingredient in the preparation of a medicament or pharmaceutical composition, wherein the active ingredient is selected from the group consisting of:
(A1) the small RNA, an RNA or DNA complementary or reverse complementary thereto, or a modified derivative thereof according to claim 1; the short hairpin RNA according to claim 5; the polynucleotide according to claim 6 or the expression vector according to claim 7; or the host cell according to claim 8;
(A2) a small RNA, MIR-2911, which has the nucleotide sequence of SEQ ID NO.54, an RNA or DNA complementary or reverse complementary thereto, or a modified derivative thereof; a precursor RNA thereof; or a vector or host cell expressing the same; and
(A3) a combination of (A1) and (A2) above;
said medicament or pharmaceutical composition is used for:
(i) preventing, treating and/or alleviating feline infectious peritonitis; and/or
(ii) inhibiting the feline infectious peritonitis virus (FIPV).
